# EUROPÄISCHE PATENTANMELDUNG

(11) **EP 3 734 085 A1**
(43) Veröffentlichungstag der Anmeldung: **04.11.2020**
(21) Anmeldenummer: 20171552.1
(22) Anmeldetag: 27.04.2020
(51) Int. Cl.: F16B 2/22, B60H 3/00, F16B 2/00

(54) **BEDUFTUNGSVORRICHTUNG UND SPREIZKLEMME FÜR EINE BEDUFTUNGSVORRICHTUNG**

(30) Priorität: 02.05.2019 CH 5862019
(71) Anmelder: Supair-Tel AG, 8152 Glattbrugg (CH)
(72) Erfinder: Guggenheim, Rudolf Heinrich, 8832 Wollerau (CH)
(74) Vertreter: Rüedi, Regula Béatrice

(57) **Zusammenfassung**

Eine Beduftungsvorrichtung (1) für ein Fahrzeug, umfasst mindestens einen Duftkörper (2) und mindestens eine am oder im Duftkörper befestigte Spreizklemme (3). Die Spreizklemme ist zur Befestigung des Duftkörpers im Fahrzeuginneren an einem Gitter (21) oder an einer Lamelle oder Lamellen (21') eines Luftauslasses (20) im Fahrzeug bestimmt, indem die Spreizklemme zwei sich in Längsrichtung der Spreizklemme und vom Duftkörper weg erstreckende und einander quer zur Längsrichtung gegenüberliegende und elastisch verformbare Klemmglieder (5, 7) aufweist, welche aus einem ersten Kunststoffmaterial gebildet sind. Einander direkt gegenüberliegende Abschnitte (8, 9) der Klemmglieder sind hingegen aus einem zweiten, weicheren Kunststoffmaterial als das erste Kunststoffmaterial gebildet. Damit wird eine gute Befestigung am Luftauslass bewirkt und dessen Verkratzen wird vermieden.

## Beschreibung

### Gebiet der Erfindung

Die Erfindung betrifft eine Beduftungsvorrichtung für ein Fahrzeug, welche Beduftungsvorrichtung mindestens einen Duftkörper und mindestens eine am oder im Duftkörper befestigte Spreizklemme umfasst, welche Spreizklemme zur Befestigung des Duftkörpers im Fahrzeuginneren an einem Gitter oder an einer Lamelle oder Lamellen eines Luftauslasses im Fahrzeug bestimmt ist, indem die Spreizklemme zwei sich in Längsrichtung der Spreizklemme erstreckende und einander quer zur Längsrichtung gegenüberliegende und elastisch verformbare Klemmglieder aufweist, welche aus einem ersten Kunststoffmaterial gebildet sind. Ferner betrifft die Erfindung eine Spreizklemme für die genannte Beduftungsvorrichtung.

### Hintergrund

Es sind Beduftungsvorrichtungen bekannt, welche einen Duftkörper umfassen und mindestens ein im oder am Duftkörper befestigtes Mittel zur Befestigung des Duftkörpers im Inneren eines Fahrzeugs an einer mit einem Gitter oder Lamellen versehenen Belüftungsöffnung des Fahrzeugs. Der Duftkörper ist ein Duftstoffe enthaltender Körper beliebiger Form. Solche Beduftungsvorrichtungen sind zum Beispiel aus WO 2017/108708 A1, WO 2015/179992 A1 und WO 2012/129709 A1 bekannt. Insbesondere kann das Mittel zur Befestigung am Gitter oder den Lamellen eine Spreizklemme sein, welche sich beim Einführen in die Belüftungsöffnung beim Einschieben in Gitteröffnungen oder zwischen Lamellen elastisch aufweitet und dann durch die Rückstellkraft der elastisch aufgeweiteten Klemme den Duftkörper am Gitter oder an der Lamelle oder den Lamellen festhält. Eine solche Klemme erlaubt auch das einfache Entfernen der Beduftungsvorrichtung, wenn die Duftstoffe aufgebraucht sind und die Beduftungsvorrichtung ersetzt werden muss.

### Darstellung der Erfindung

Der Erfindung liegt die Aufgabe zu Grunde eine verbesserte Beduftungsvorrichtung zu schaffen, welche eine verbesserte Befestigung ermöglicht und Beschädigungen am Fahrzeug vermeidet.

Diese Aufgabe wird bei der Beduftungsvorrichtung der eingangs genannten Art dadurch gelöst, dass einander direkt gegenüberliegende Abschnitte der Klemmglieder aus einem zweiten, weicheren Kunststoffmaterial als das erste Kunststoffmaterial gebildet sind.

Dies ermöglicht einerseits eine bessere Haftung der Spreizklemme am Gitter oder an der Lamelle bzw. den Lamellen, indem der weichere Kunststoff einen Formschluss mit der Form des Gitters bzw. der Lamellen ermöglicht, da sich der weichere Kunststoff unter dem Einfluss der Klemmkraft der Spreizklemme etwas verformen kann. Ferner vermeidet der weichere Kunststoff eine Beschädigung des Gitters bzw. der Lamellen, insbesondere ein Verkratzen solcher Teile, da diese regelmässig aus einem härteren Kunststoff gefertigt sind als der weichere Kunststoff der Spreizklemme. Bei der erfindungsgemässen Beduftungsvorrichtung kann der Duftkörper ein beliebiger dem Fachmann bekannter Duftkörper sein, wie er in Fahrzeugen verwendet wird.

Bevorzugt ist der erste Kunststoff ein thermoplastischer Kunststoff, insbesondere Acrylnitril-Butadien-Styrol (ABS) und der zweite Kunststoff ein thermoplastisches Elastomer bzw. ein thermoplastischer gummielastischer Kunststoff. Mit einer solchen Kunststoffkombination kann die Spreizklemme der Beduftungsvorrichtung in einem Schritt durch Mehrkomponenten-Spritzgiessen und damit kostengünstig hergestellt werden.

Weiter ist es bevorzugt, dass mindestens eines der Klemmglieder einen in Richtung des Duftkörpers zurückgebogenen Klemmteil aufweist, auf welchem ein Abschnitt des zweiten Kunststoffmaterials vorgesehen ist. Auf diese Weise kann die Klemmkraft erhöht werden bzw. es kann auch eine gute Befestigung z.B. an dünnen Lamellen erreicht werden und in Kombination mit dem weicheren Kunststoff am zurückgebogenen Klemmenteil ergibt sich eine besonders gute Befestigung und auch eine besonders gute Vermeidung des Beschädigens bzw. Verkratzens der Lüftungselemente des Fahrzeugs.

Ferner ist es bevorzugt, dass die Spreizklemme einen zur Befestigung im Duftkörper ausgestalteten Befestigungsabschnitt mit mindestens einem Rückhalteelement und mit einem Auflageteller für den Duftkörper versehen ist. Damit kann eine gute Befestigung im Duftkörper erreicht werden, so dass bei der Handhabung der Beduftungsvorrichtung mit der gut am Lüftungselement des Fahrzeugs klemmenden Spreizklemme keine Gefahr besteht, dass sich die Spreizklemme vom Duftkörper löst.

Ferner liegt der Erfindung die Aufgabe zu Grunde eine verbesserte Spreizklemme für eine Beduftungsvorrichtung der eingangs genannten Art zu schaffen.

Diese Aufgabe wird mit einer Spreizklemme gemäss Anspruch 5 gelöst. Es ergeben sich damit die bereits vorstehend bei der Beduftungsvorrichtung genannten Vorteile.

### Kurze Beschreibung der Zeichnungen

Weitere Ausgestaltungen, Vorteile und Anwendungen der Erfindung ergeben sich aus den abhängigen Ansprüchen und aus der nun folgenden Beschreibung anhand der Figuren. Dabei zeigen:
Figur 1 eine Schnittansicht einer Ausführungsform einer Beduftungsvorrichtung gemäss der Erfindung mit einem nur teilweise und schematisch dargestellten Duftkörper; und
Figur 2 eine schaubildliche Darstellung einer Klemme gemäss der Erfindung.

### Weg(e) zur Ausführung der Erfindung

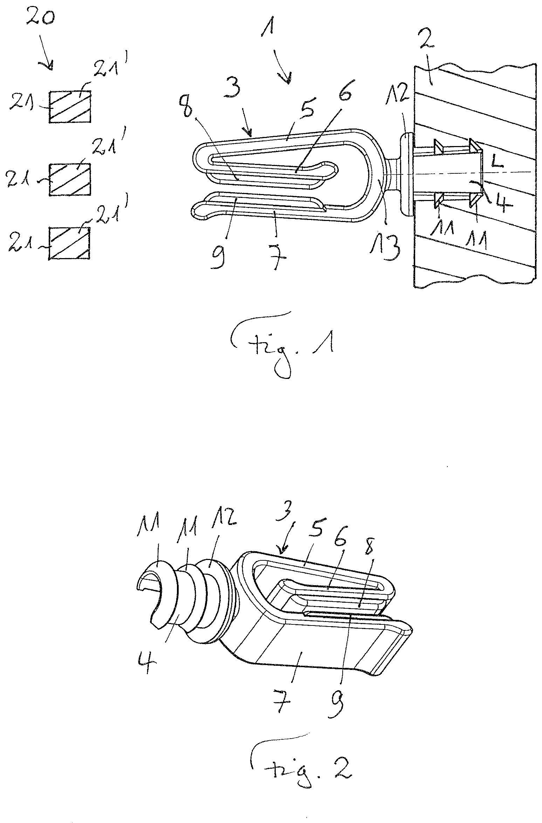

Figur 1 zeigt eine Schnittdarstellung einer Beduftungsvorrichtung 1, welche einen Duftkörper 2 und eine in diesem befestigte Spreizklemme 3 umfasst und Figur 2 zeigt die Spreizklemme 3 alleine. Der Duftkörper kann ein beliebiger Duftkörper sein, wie in Automobilen zur Anwendung kommt, also ein Duftstoffe enthaltender Kunststoffkörper, ein mit Duftstoffen getränkter Körper aus einem porösen Material oder ein Behälter, welcher flüssigen Duftstoff enthält, der über Öffnungen im Behälter nach und nach abgegeben wird. Der Duftkörper wird mittels der Spreizklemme 3 im Fahrzeug an einem nur angedeuteten Luftauslass 20 des Fahrzeugs befestigt. Der Luftauslass weist ein Gitter 21 oder Lamellen 21' auf, wie dies dem Fachmann bekannt und in Figur 1 nur angedeutet ist. Wird die Spreizklemme 3 mit ihren Klemmgliedern in das Gitter oder zwischen den Lamellen eingeführt, so spreizen sich die elastisch verformbaren Klemmglieder auf und halten sich durch ihre Rückstellkraft an dem Gitter oder den Lamellen fest, womit der Duftkörper 2 bzw. die ganze Beduftungsvorrichtung 1 im Fahrzeug befestigt ist. Ist der Duftstoff im Duftkörper, der im Gebrauch von der aus der Lüftung des Fahrzeugs austretenden Luftstrom umströmt wird, verbraucht, so kann die Spreizklemme wieder aus dem Gitter oder von den Lamellen abgezogen werden. Im gezeigten Beispiel ist die Spreizklemme der Beduftungsvorrichtung im Duftkörper mittels eines Befestigungsabschnitts 4 befestigt. Die Spreizklemme kann aber auch nur aussen am Duftkörper befestigt sein.

Die Spreizklemme weist eine Längsrichtung auf, welche in der Figur durch eine gestrichelte Längsachse L angedeutet ist. Die Klemmglieder 5 und 7 erstrecken sich in Längsrichtung und vom Duftkörper 2 bzw. vom Befestigungsabschnitt 4 weg, so dass sie zur Einführung in die Lüftungsöffnung des Fahrzeugs geeignet sind. Die Klemmglieder liegen einander quer zur Längsrichtung gesehen gegenüber, so dass sie eine Klemme oder Klammer oder einen Clip bilden. Diese Begriffe werden hier vom Fachmann als gleichbedeutend verstanden und erklären sich im Hinblick auf die Figuren und die erläuterte Funktion von selber.

In Figur 1 ist eine bevorzugte Ausführung dargestellt, bei welcher die Spreizklemme 3 der Beduftungsvorrichtung 1 ein Klemmglied 5 aufweist, welches einen in Richtung auf den Duftkörper 2 bzw. in Richtung auf den Befestigungsabschnitt 4 der Spreizklemme 3 zurückgebogenen Klemmteil 6 aufweist. Das andere Klemmglied 7 ist nicht so ausgeführt und erstreckt sich in einfacher Form ohne Rückbiegung in Längsrichtung. Auch das Klemmglied 5 könnte so ausgeführt sein wie das Klemmglied 7 bzw. ohne zurückgebogenen Klemmteil. Es können auch beide Klemmglieder 5 und 7 mit einem zurückgebogenen Klemmteil versehen sein. Im gezeigten Beispiel sind die beiden Klemmglieder 5, 7 zusammen am Befestigungsabschnitt 4 angeordnet, bzw. vor dem Befestigungsabschnitt im Bereich 13 zusammengeführt. Die Klemmglieder 5, 7 könnten aber auch einzeln vom Befestigungsabschnitt 4 ausgehen.

Die quer zur Längsrichtung einander gegenüberliegenden und elastisch verformbaren Klemmglieder 5, 7 sind aus einem ersten Kunststoffmaterial gebildet, während die einander direkt gegenüberliegenden Abschnitte 8, 9 der Klemmglieder 5 (mit dem Klemmteil 6) und 7 aus einem zweiten, weicheren Kunststoffmaterial als das erste Kunststoffmaterial gebildet sind. Bevorzugt ist auch der Befestigungsabschnitt 4 der Spreizklemme aus dem ersten Kunststoff gebildet. Der erste und der zweite Kunststoff sind bevorzugt thermoplastische Kunststoffe, so dass eine Herstellung der Spreizklemme 3 durch Mehrkomponenten-Spritzgiessen möglich ist. Als erster Kunststoff ist der Acrylnitril-Butadien-Styrol (ABS) bevorzugt und der zweite Kunststoff ist ein thermoplastisches Elastomer, also ein gummielastischer Kunststoff, der weicher als der erste Kunststoff ist. Der zur Befestigung im Duftkörper 2 ausgestalteten Befestigungsabschnitt 4, welcher ebenfalls aus dem ersten Kunststoff gebildet ist, weist bevorzugt mindestens ein Rückhalteelement 11 auf, welches ein gutes Haften der Spreizklemme 3 der Beduftungsvorrichtung im Duftkörper 2 der Beduftungsvorrichtung ergibt. Bevorzugt ist ferner ein Auflageteller 12 für die Aussenseite des Duftkörpers 2 an der Spreizklemme 3 vorgesehen. Die Spreizklemme 3 ist in der Regel ein Vollkörper aus den beiden Kunststoffen mit einem hohlzylindrischen Befestigungsabschnitt 4 und die Darstellungen von Figur 1 und Figur 2 sind so zu verstehen.

Während in der vorliegenden Anmeldung bevorzugte Ausführungen der Erfindung beschrieben sind, ist klar darauf hinzuweisen, dass die Erfindung nicht auf diese beschränkt ist und in auch anderer Weise innerhalb des Umfangs der folgenden Ansprüche ausgeführt werden kann.

## Patentansprüche

1. Beduftungsvorrichtung (1) für ein Fahrzeug, welche Beduftungsvorrichtung mindestens einen Duftkörper (2) und mindestens eine am oder im Duftkörper befestigte Spreizklemme (3) umfasst, welche Spreizklemme zur Befestigung des Duftkörpers im Fahrzeuginneren an einem Gitter (21) oder an einer Lamelle oder Lamellen (21') eines Luftauslasses (20) im Fahrzeug bestimmt ist, indem die Spreizklemme zwei sich in Längsrichtung der Spreizklemme und vom Duftkörper weg erstreckende und einander quer zur Längsrichtung gegenüberliegende und elastisch verformbare Klemmglieder (5, 7) aufweist, welcher aus einem ersten Kunststoffmaterial gebildet sind, **dadurch gekennzeichnet, dass** einander direkt gegenüberliegende Abschnitte (8, 9) der Klemmglieder aus einem zweiten, weicheren Kunststoffmaterial als das erste Kunststoffmaterial gebildet sind.

2. Beduftungsvorrichtung nach Anspruch 1, wobei der erste Kunststoff ein thermoplastischer Kunststoff ist, insbesondere Acrylnitril-Butadien-Styrol (ABS) und der zweite Kunststoff ein thermoplastisches Elastomer ist.

3. Beduftungsvorrichtung nach Anspruch 1 oder 2, wobei mindestens eines der Klemmglieder (5) einen in Richtung des Duftkörpers (2) zurückgebogenen Klemmteil (6) aufweist, an welchem einer der Abschnitte (8) des zweiten Kunststoffmaterials vorgesehen ist.

4. Beduftungsvorrichtung nach einem der Ansprüche 1 bis 3, wobei die Spreizklemme (3) einen zur Befestigung im Duftkörper (2) ausgestalteten Befestigungsabschnitt (4) aufweist, welcher ebenfalls aus dem ersten Kunststoff gebildet ist und insbesondere mit mindestens einem Rückhalteelement (11) und mit einem Auflageteller (12) für die Aussenseite des Duftkörpers versehen ist.

5. Spreizklemme (3) für eine Beduftungsvorrichtung nach einem der Ansprüche 1 bis 4, welche Spreizklemme zur Befestigung des Duftkörpers im Fahrzeuginneren an einem Gitter (21) oder an einer Lamelle oder Lamellen (21') eines Luftauslasses (20) im Fahrzeug bestimmt ist, indem die Spreizklemme zwei sich in Längsrichtung der Spreizklemme und vom Duftkörper weg erstreckende und einander quer zur Längsrichtung gegenüberliegende und elastisch verformbare Klemmglieder (5, 7) aufweist, welche aus einem ersten Kunststoffmaterial gebildet sind, **dadurch gekennzeichnet, dass** einander direkt gegenüberliegende Abschnitte (8, 9) der Klemmglieder aus einem zweiten, weicheren Kunststoffmaterial als das erste Kunststoffmaterial gebildet sind.

6. Spreizklemme nach Anspruch 5, wobei der erste Kunststoff ein thermoplastischer Kunststoff ist, insbesondere Acrylnitril-Butadien-Styrol (ABS) und der zweite Kunststoff ein thermoplastisches Elastomer ist.

7. Spreizklemme nach einem der Ansprüche 5 oder 6, wobei die Spreizklemme einen zur Befestigung im Duftkörper bestimmten Befestigungsabschnitt (4) aufweist, welcher mit mindestens einem Rückhalteelement (11) und mit einem Auflageteller (12) für den Duftkörper versehen ist.

8. Spreizklemme nach Anspruch 7, wobei mindestens eines der Klemmglieder (5) einen in Richtung des Befestigungsabschnitts (4) zurückgebogenen Klemmteil (6) aufweist, an welchem ein Abschnitt (8) des zweiten Kunststoffmaterials vorgesehen ist.
